# EUROPÄISCHE PATENTSCHRIFT

(11) **EP 3 416 596 B1**
(45) Veröffentlichungstag und Bekanntmachung des Hinweises auf die Patenterteilung: **01.04.2020**
(21) Anmeldenummer: 17708447.2
(22) Anmeldetag: 16.02.2017
(51) Int. Cl.: A61F 2/966, A61F 2/95

(54) **STENT-DELIVERY-SYSTEM**
STENT DELIVERY SYSTEM
SYSTÈME DE DISTRIBUTION DE STENT

(30) Priorität: 19.02.2016 DE 102016102990; 18.08.2016 DE 102016115351
(43) Veröffentlichungstag der Anmeldung: 26.12.2018
(73) Patentinhaber: Joline GmbH & Co. KG, 72379 Hechingen (DE)
(72) Erfinder: MAIER, Jan, 72116 Moessingen (DE); EISOLD, Gerd, 72415 Grosselfingen (DE)
(74) Vertreter: DREISS Patentanwälte PartG mbB
(86) Internationale Anmeldenummer: PCT/EP2017/053490
(87) Internationale Veröffentlichungsnummer: WO 2017/140772

(56) Entgegenhaltungen:
- DE-A1-102006 004 123
- DE-A1-102013 015 896
- US-A1- 2007 270 932
- US-A1- 2014 135 909
- US-A1- 2014 180 380

## Beschreibung

Die Erfindung betrifft ein System zum Einführen eines komprimierten, selbstexpandierbaren Stents durch eine an einem Blutgefäß angeordnete Schleuse hindurch in das Blutgefäß und zur Freisetzung des Stents in dem Blutgefäß, mit einem Katheter umfassend einen Innenschlauch, einen Mittelschlauch und einen Außenschlauch, wobei sich der Innenschlauch durch den Mittelschlauch erstreckt und sich der Mittelschlauch durch den Außenschlauch erstreckt. Dabei ist der komprimierte Stent in einem distalen Endabschnitt des Katheters radial um den Innenschlauch verlaufend zwischen dem Innenschlauch und dem Außenschlauch radial komprimiert angeordnet, wobei der Mittelschlauch distal am proximalen Ende des Stents angeordnet ist bzw. am proximalen Ende des Stents endet. Ferner ist im proximalen Bereich des Katheters zur Freisetzung des Stents eine Betätigungseinheit mit einer Antriebseinheit vorgesehen.

Ein derartiges System ist aus der DE 10 2013 015 896 A1 vorbekannt. Weitere Merkmale des Oberbegriffs des Anspruchs 1 sind aus der US 2014/0135909 A1 vorbekannt.

Aus dem Stand der Technik sind zudem unterschiedliche Arten von Stents vorbekannt. Insbesondere sind lasergeschnittene Stents bereits seit längerem bekannt. Diese passen sich einem Lumen allerdings nur bedingt an und es hat sich herausgestellt, dass diese dazu neigen, in einem Lumen nach einigen Jahren zu brechen. Deswegen wurden Flechtstents entwickelt, welche nicht nur flexibler sind, sondern auch noch nach Jahren des Einsatzes in einem Lumen nicht brechen und kostengünstig herstellbar sind. Derartige Flechtstents dehnen sich bei der Freisetzung in einem Lumen in radialer Richtung aus und weiten somit ein Lumen auf. Allerdings ändert sich bei der Freisetzung die Position der Stents insbesondere auf Grund der Änderung der Länge und des Durchmessers. Somit sind diese schwieriger zu positionieren als lasergeschnittene Stents.

Ferner sind aus dem Stand der Technik verschiedene Einführsysteme für insbesondere geflochtene, selbstexpandierbare Stents vorbekannt. In der Regel handelt es sich dabei um Kathetersysteme, mithilfe derer der Stent an seinen Einsatzort geschoben und daraufhin dadurch expandiert wird, dass der Stent aus dem Katheter freigesetzt wird. Mit solchen Einführsystemen werden Gefäßstents in Blutgefäße implantiert, welche aufgrund einer Krankheit oder ähnlichem verletzt oder in ihrem Lumen verschlossen oder verengt sind, wodurch die Gefäße in ihrer Funktion stark beeinträchtigt sind.

Zur Implantation wird der Stent in der Regel in einem Katheter radial zusammengedrückt, sodass sich seine Länge deutlich verlängert, sodass er relativ einfach in das Gefäß eingeführt werden kann. Der Katheter wird dann durch die sogenannte Schleuse in das Blutgefäß eingeführt. Die Schleuse ist ein Katheter, über den bei Bedarf andere Katheter oder Instrumente in einen Patienten zu medizinischen Zwecken eingeführt werden können. Die Seldingertechnik oder Seldinger-Methode stellt dabei eine Methode zur Punktion von Blutgefäßen zum Zweck der Katheterisierung (Anbringen der Schleuse) dar.

Handelt es sich bei dem Stent um einen selbstexpandierenden Stent, so expandiert dieser sich aufgrund seiner Elastizität bzw. Federwirkung bei einer Freisetzung selbst wieder in seine ursprüngliche Form und spannt dabei seine Mantelfläche auf, sodass diese sich in dem Blutgefäß anlegt beziehungsweise das Lumen des Blutgefäßes aufweitet oder aufrechterhält. Dies macht auch hocherfahrenen Anwendern eine millimetergenaue Positionierung des Stents schwierig. Eine exakte Positionierung ist aus medizinischer Sicht jedoch unbedingt erwünscht bzw. notwendig. Die Lage des Stents kann dabei insbesondere über Röntgenmarker kontrolliert werden.

Der folgenden Erfindung liegt die Aufgabe zugrunde, eine genauere Platzierung des Stents samt einer vorteilhaften Stentexpansion zu ermöglichen.

Diese Aufgabe wird mit einem System mit den Merkmalen des Anspruchs 1 gelöst. Es ist folglich vorgesehen, dass zwischen der Antriebseinheit und der Schleuse ein Verbindungsmittel vorgesehen ist. Dabei wirkt die Antriebseinheit derart mit dem Mittelschlauch und dem Verbindungsmittel zusammen, dass bei deren Betätigung zum einen der Mittelschlauch in distaler Richtung verschoben wird und zum anderen die Betätigungseinheit samt daran befestigtem Außenschlauch in proximaler Richtung verschoben wird. Ferner wirkt der Innenschlauch derart mit dem Mittelschlauch und/oder dem Verbindungsmittel zusammen, dass dieser zumindest bedingt relativ zur Betätigungseinheit und damit zum Außenschlauch verlagert wird. Denkbar ist allerdings auch, dass der Innenschlauch mit der Antriebseinheit so zusammenwirkt, dass dieser relativ zur Betätigungseinheit und damit zum Außenschlauch verlagert wird.

Bei dem selbstexpandierbaren Stent kann es sich dabei insbesondere um einen selbstexpandierbaren, geflochtenen Stent oder um einen mittels anderer Verfahren hergestellten Stent aus Metalldraht oder Kunststofffaden handeln. Insbesondere bei geflochtenen Stents besteht die Problematik, dass sich diese bei der Freisetzung sehr stark ausdehnen und damit nach der Freisetzung nicht ihre Bestimmungsposition einnehmen.

Das Verbindungsmittel kann einerseits starr ausgebildet sein oder eine Anordnung starrer, gegeneinander bewegbarer Verbindungsmittelabschnitte umfassen. So ist denkbar, dass das Verbindungsmittel als Teleskopstange oder Teleskoprohr oder als Zahnstange ausgebildet ist. Andererseits ist insbesondere auch denkbar, dass das Verbindungsmittel elastisch biegbar - beispielsweise als Draht oder als flexible Zahnstange - ausgebildet ist oder einen elastisch biegbaren Verbindungsmittelabschnitt umfasst.

Dadurch, dass vorgesehen ist, dass die Antriebseinheit so ausgebildet ist, dass bei deren Betätigung zum einen der Mittelschlauch in distaler Richtung verschoben wird und zum anderen die Betätigungseinheit und der daran angeordnete Katheter in proximaler Richtung verschoben werden, kann erreicht werden, dass sich die Bestimmungsposition des distalen Endes des Stents während der Freisetzung nicht oder nur geringfügig ändert. Die Freisetzung des Stents erfolgt also dadurch, dass der Mittelschlauch das proximale Ende des komprimierten Stents nach vorne, also in distale Richtung schiebt, wohingegen die Betätigungseinheit in proximaler Richtung bewegt wird, wobei auch der daran angeordnete Katheter sowie der am Katheter angeordneten Stent in proximaler Richtung bewegt werden.

Gleichzeitig behält das distale Ende des Innenschlauchs zumindest bedingt seine Position, wenn der Innenschlauch mit dem Verbindungsmittel bewegungsgekoppelt, da die Betätigungseinheit dann zumindest bedingt relativ zum Innenschlauch verschiebbar ist. Regelmäßig weist der Innenschlauch am distalen Ende eine Spitze auf, welche den Katheter dicht verschließt. Dadurch, dass beim Freisetzen des Stents die Betätigungseinheit und damit der Außenschlauch in proximaler Richtung bewegt werden, während der Mittelschlauch in distaler Bewegungsrichtung bewegt wird und gleichzeitig jedenfalls zu Beginn der Freisetzung der Innenschlauch seine distale Endposition beibehält, wird der Stent nach und nach aus dem Katheter freigesetzt.

Ist der Innenschlauch demgegenüber mit dem Mittelschlauch bewegungsgekoppelt, so bewegt sich dieser mit dem Mittelschlauch in distaler Richtung, während der Außenschlauch in proximaler Richtung bewegt wird. Auch hierdurch wird der Stent nach und nach freigesetzt.

Zudem verhindert das Verbindungsmittel auch eine unkontrollierte Bewegung der Betätigungseinheit in proximaler Richtung bei Freisetzung des Stents. In dem Stand der Technik DE 10 2013 015 896 A1 ist demgegenüber kein Verbindungsmittel zwischen Schleuse und Antriebseinheit vorgesehen, sodass insbesondere auf Grund von Reibungskräften zwischen Außenschlauch und Mittelschlauch, Außenschlauch und Schleuse sowie Außenschlauch und Blutgefäß die Betätigungseinheit beim Freisetzen des Stents einem Moment in proximaler Richtung unterliegt, sodass eine Bedienperson einer Bewegung der Betätigungseinheit in proximaler Richtung entgegenwirken muss.

Die Bewegung des Mittelschlauchs und der Betätigungseinheit mit dem daran angeordneten Katheter kann dabei synchron oder abwechselnd um dieselbe oder unterschiedliche Distanz erfolgen. Vorzugsweise ist ein Übersetzungsverhältnis vorhanden, sodass die Bewegung um unterschiedliche Distanzen erfolgt.

Insgesamt wird mittels des erfindungsgemäßen Systems erreicht, dass nach der Freisetzung des Stents, nicht nur das distale Ende des Stents, sondern auch das proximale Ende des Stents seine Bestimmungsposition einnimmt und damit der gesamte Stent nach der Freisetzung seine Bestimmungsposition einnimmt. Die Bestimmungsposition kann dabei innerhalb eines vorgegebenen Bereichs, insbesondere zwischen 0 und 5 mm, liegen. Folglich nimmt der Stent nach der Freisetzung seine Solllänge und damit seine Gesamtbestimmungsposition ein. Zudem wird durch das Bewegen der Betätigungseinheit relativ zum Innenschlauch auch eine vorteilhafte Reibungsreduktion zwischen den einzelnen Katheterschläuchen erzielt.

Eine vorteilhafte Weiterbildung der Erfindung sieht vor, dass der Innenschlauch mit dem Mittelschlauch bewegungsgekoppelt ist. Dabei ist der komprimierte Stent derart am Innenschlauch kraftschlüssig angeordnet, dass dieser beim Verschieben des Mittelschlauchs und damit des Stents in distaler Richtung ebenfalls in distaler Richtung verschoben wird.

Hierbei wird der Innenschlauch folglich zusammen mit dem Mittelschlauch in distaler Richtung verschoben, so dass eine Freisetzung des Stents ermöglicht wird.

Dabei kann insbesondere vorgesehen sein, dass die Betätigungseinheit eine Bewegungsbegrenzung für den Innenschlauch aufweist, sodass eine Bewegung des Innenschlauchs relativ zur Betätigungseinheit lediglich bedingt ermöglicht ist.

Somit kann insbesondere vorgesehen sein, dass der Innenschlauch zu Beginn der Stentfreisetzung in distaler Richtung bewegt wird, um zu ermöglichen, dass der Katheter so geöffnet wird, dass der Stent sich aus dem Katheter herausbewegen kann. In diesem Zusammenhang kann insbesondere vorgesehen sein, dass der Innenschlauch im proximalen Bereich in einem Führungsschlauch mit einem Spülanschluss (Luer-Anschluss) geführt ist und das proximale Ende des Innenschlauchs am Spülanschluss befestigt ist. Der Führungsschlauch kann hierbei in der Betätigungseinheit enden und ein Begrenzungsmittel aufweisen, das in einer Führung in der Betätigungseinheit geführt ist. Ferner kann die Betätigungseinheit einen dazu komplementären Anschlag aufweisen, so dass eine Bewegung des Innenschlauchs lediglich begrenzt möglich ist, nämlich bis das Begrenzungsmittel des Führungsschlauchs den Anschlag erreicht. Sobald der Anschlag erreicht ist, wird der Innenschlauch ebenfalls in proximaler Richtung bewegt.

Insbesondere kann hierbei eine Bewegung des Innenschlauchs in distaler Richtung zwischen 50 bis 250 mm, insbesondere 200 mm, möglich sein. Insgesamt kann der komprimierte Stent eine Länge von 100 bis 600 mm, insbesondere 570 mm, aufweisen. Folglich kann eine Bewegungskopplung des Innenschlauchs mit dem Mittelschlauch für die ersten 200 mm Bewegungsweg des Mittelschlauchs in distaler Richtung stattfinden. Für die weiteren 300 bis 400 mm, insbesondere 370 mm, wird lediglich der Mittelschlauch in distaler Richtung bewegt, während der Innenschlauch zusammen mit dem Außenschlauch aufgrund der Bewegung der Betätigungseinheit in proximaler Richtung bewegt werden.

Andererseits ist auch denkbar, dass der Innenschlauch am Verbindungsmittel befestigt ist, sodass die Betätigungseinheit samt Außenschlauch beim Verschieben derselben in proximaler Richtung relativ zum Innenschlauch bewegt wird.
Folglich kann das distale Ende des Innenschlauchs seine Position beibehalten, da die Betätigungseinheit samt Außenschlauch aufgrund des Zusammenwirkens mit dem Verbindungsmittel in proximaler Richtung bewegt wird, während der Innenschlauch aufgrund der Verbindung mit dem Verbindungsmittel seine Position beibehält.

Besonders bevorzugt ist, wenn das Verbindungsmittel einen ersten Abschnitt und einen zweiten Abschnitt umfasst. Dabei kann der erste Abschnitt an der Schleuse befestigt sein, während der zweite Abschnitt am Innenschlauch befestigt sein kann und mit der Antriebseinheit zusammenwirken kann. Ferner können die beiden Abschnitte in einem ersten Betriebszustand relativ zueinander verlagerbar sein und in einem zweiten Betriebszustand aneinander befestigt sein.

Folglich kann das Verbindungsmittels teleskopisch ausgebildet sein und insbesondere einen als Draht ausgebildeten ersten Abschnitt aufweisen, der im Bereich der Betätigungseinheit durch einen als Schlauch ausgebildeten zweiten Abschnitt geführt ist. Die beiden Abschnitte können dabei insbesondere beispielsweise durch eine Spannvorrichtung gegeneinander verspannbar sein. Die Spannvorrichtung kann insbesondere im distalen Bereich der Betätigungseinheit vor der Betätigungseinheit angeordnet sein und manuell betätigbar sein. Hierdurch ergibt sich der Vorteil, dass die Abschnitte im ersten Betriebszustand gegeneinander verlagerbar sind und somit der anfängliche Abstand der Betätigungseinheit zur Schleuse eingestellt werden kann, ohne dass hierbei eine Betätigung des Verlagerungselements bzw. des zweiten Abschnitts davon durch die Antriebseinheit stattfände und somit auch keine Verlagerung des Innenschlauchs relativ zur Betätigungseinheit stattfände.

Besonders bevorzugt ist dabei, wenn das Zusammenwirken der Antriebseinheit mit dem Mittelschlauch aufhebbar ist. Alternativ oder zusätzlich kann auch das Zusammenwirken mit dem Verbindungsmittel aufhebbar sein.

Vorteilhafterweise kann sowohl das Zusammenwirken der Antriebseinheit mit dem Mittelschlauch als auch mit dem Verbindungsmittel abhängig oder insbesondere auch unabhängig voneinander aufhebbar sein. Hierdurch wird eine größtmögliche Flexibilität für die Bedienperson gewährleistet. Insbesondere kann die Bedienperson damit die Betätigungseinheit positionieren, bevor der Stent freigesetzt wird, ohne dass Relativbewegungen der Katheterschläuche relativ zueinander ausgelöst werden.

Besonders bevorzugt ist weiter, wenn die Antriebseinheit an einem Lagerabschnitt angeordnet ist, wobei der Lagerabschnitt verschwenkbar ist, sodass das Zusammenwirken der Antriebseinheit mit dem Mittelschlauch und/oder dem Verbindungsmittel aufhebbar ist.
Insbesondere kann der Lagerabschnitt hierbei um eine Schwenkachse im distalen Bereich der Betätigungseinheit verschwenkbar sein. Dabei kann die Antriebseinheit insbesondere eine Drehwelle umfassen, wobei die Drehwelle Mittel zum Zusammenwirken mit dem Verbindungsmittel bzw. dem Mittelschlauch aufweisen kann. Bei diesen Mitteln kann es sich insbesondere um Zahnräder oder Ritzel handeln. Hierbei kann die Drehwelle insbesondere am Lagerabschnitt drehbar gelagert sein. Dabei kann der proximale Abschnitt des Lagerabschnitts am proximalen Bereich der Betätigungseinheit der Betätigungseinheit insbesondere mittels einer Rast- oder Schnappverbindung lösbar befestigbar sein.

Vorteilhafterweise ist ein mit dem Innenschlauch bewegungsgekoppeltes Verlagerungselement vorhanden. Dabei kann das Verlagerungselement in einer ersten Lage so an der Betätigungseinheit angeordnet ist, dass der Lagerabschnitt so verschwenkt ist, dass das Zusammenwirken der Antriebseinheit mit dem Mittelschlauch und/oder dem Verbindungsmittel aufgehoben ist. In einer zweiten Lage kann das Verlagerungselement so entlang der Betätigungseinheit in distaler Richtung verlagert sein, dass der Lagerabschnitt verschwenkbar ist.

Somit ist eine Verlagerung des Mittelschlauchs und/oder des Verbindungsmittels erst dann möglich, wenn die Verlagerungsrichtung in distaler Richtung verschoben ist und somit ein Verschwenken des Lagerabschnitts freigibt.

Somit wird der Innenschlauch zunächst in distaler Richtung durch Verlagerung des Verlagerungselements relativ zu Mittel- und Außenschlauch verlagert, bevor mit der eigentlichen Stentfreisetzung begonnen wird. Hierdurch kann sichergestellt werden, dass der Stent ausreichend Raum zur Expansion aufweist.

Insbesondere weist der Innenschlauch hierbei am distalen Ende eine Spitze auf, wobei die Spitze den Katheter fluiddicht verschließt, wenn das Verlagerungselement in der ersten Lage ist, und wobei der Katheter geöffnet ist, wenn das Verlagerungselement in der zweiten Lage ist.

Somit kann der Katheter in der fluiddichten Lage gespült werden und sodann in der zweiten Lage geöffnet sein, um eine Stentexpansion sicher zu ermöglichen.

Eine besonders bevorzugte Weiterbildung der Erfindung ergibt sich daraus, dass der Lagerabschnitt einen Bügel mit einem Anschlag aufweist, wobei das Verlagerungselement in der zweiten Lage am Anschlag anliegt, und sodann der Lagerabschnitt verschwenkbar ist.

Somit wird gewährleistet, dass der Innenschlauch und damit die distale Spitze des Innenschlauchs lediglich um einen vorbestimmten Weg, insbesondere 15 bis 25 mm verlagert wird.
Eine besonders bevorzugte Weiterbildung der Erfindung sieht vor, dass das proximale Ende des Innenschlauchs an einem Führungsschlauch mit einem Spülanschluss befestigt ist, wobei der Führungsschlauch das Verlagerungselement aufweist. Der Führungsschlauch kann hierbei im proximalen Bereich der Betätigungseinheit enden.

Besonders bevorzugt ist ferner, wenn die Betätigungseinheit ein Drückelement aufweist, das den Mittelschlauch und/oder das Verbindungsmittel zur Bewegungskopplung gegen die Antriebseinheit drückt.

Das Drückelement kann hierbei insbesondere als Blattfeder, oder auch als Spiralfeder in Verbindung mit einem Kugellager, insbesondere einem Rillenkugellager, ausgebildet sein. Hierdurch wird eine sichere Kopplung des Mittelschlauchs mit der Antriebseinheit und/oder dem Verbindungsmittel mit der Antriebseinheit gewährleistet. Dies ist insbesondere dann von Vorteil, wenn die Antriebseinheit unmittelbar mit dem Mittelschlauch und/oder dem Verbindungsmittel zusammenwirkt. Insbesondere können der Mittelschlauch und/oder das Verbindungsmittel hierbei unter Vorspannung gegen die Antriebseinheit gedrückt sein. Denkbar wäre auch, dass der Bügel des Lagerabschnitts als Drückelement wirkt.

Vorteilhafterweise ist das Drückelement verschwenkbar, sodass das Zusammenwirken der Antriebseinheit mit dem Mittelschlauch und/oder dem Verbindungsmittel aufhebbar ist.

Vorzugsweise weist das Verbindungsmittel an seinem distalen Ende einen Befestigungsabschnitt auf, mittels welchem das Verbindungsmittel an der Schleuse befestigt ist. Hierbei kann der Befestigungsabschnitt einen die Schleuse durchgreifenden Katheterführungsabschnitt aufweisen, in dem der Katheter geführt ist.

Das Innere des Katheterführungsabschnitts aber auch der gesamte Befestigungsabschnitt kann dabei zur Reibungsreduzierung zwischen Befestigungsabschnitt, Schleuse und Katheter aus POM oder PTFE bestehen oder dieses Material umfassen. Auch andere reibungsreduzierende Beschichtungen wären denkbar.

Besonders bevorzugt ist zwischen der Schleuse und dem Befestigungsabschnitt ein um das Verbindungsmittel und/oder den Katheter verlaufendes Stabilisierungselement, insbesondere eine Spiralfeder oder ein geflochtenes Element, zur Verhinderung des Durchbiegens des Verbindungsmittels und/oder des Katheters vorgesehen. Das Stabilisierungselement wirkt folglich insbesondere als Biege- bzw. Knickschutz für den Katheter aber auch für das Verbindungsmittel.

Insbesondere ist im Zusammenhang mit der Erfindung auch denkbar, dass ein Zurückziehen des lediglich teilweise freigesetzten Stents in den Katheter ermöglicht ist, in dem die Antriebsrichtung der Antriebseinheit umgekehrt wird. Insbesondere hier besteht die Gefahr, dass der zwischen Schleuse und Betätigungseinheit liegende Katheterabschnitt durch Reibungskräfte zwischen Katheter und Schleuse geknickt oder gebogen wird. Das Stabilisierungselement ermöglicht hier in vorteilhafterweise einen Knickschutz.

Weitere Einzelheiten und vorteilhafte Ausgestaltungen der Erfindung sind der nachfolgenden Beschreibung zu entnehmen, anhand derer die Erfindung näher beschrieben und erläutert wird.

Es zeigen:
- Figur 1:: a)Eine schematische perspektivische Darstellung eines erfindungsgemäßen Systems gemäß einer ersten Ausführungsform; b) schematische geschnittene Seitenansicht eines Teils des Systems im Bereich der Schleuse; c) Darstellung gemäß a), jedoch mit ausgefahrenem Verbindungsmittel;
- Figur 2: eine schematische geschnittene Ansicht des Systems gemäß Figur 1a mit einer ersten Antriebsvariante (a)) und einer zweiten Antriebsvariante (b));
- Figur 3: eine Explosionsdarstellung der Betätigungseinheit des Systems gemäß Figur 1a;
- Figur 4: eine perspektivische Ansicht des proximalen Bereichs der Betätigungseinheit in einer ersten Variante (a)) einer zweiten Variante (b));
- Figur 5: a)eine schematische perspektivische Teilexplosionsdarstellung eines erfindungsgemäßen Systems gemäß einer zweiten Ausführungsform; b) schematische Seitenansicht des proximalen Bereichs des Systems;
- Figur 6: a)eine schematische Seitenansicht eines erfindungsgemäßen Systems gemäß einer dritten Ausführungsform; b) schematische Seitenansicht eines Teils des Systems im Bereich der Schleuse; c) schematische Seitenansicht des proximalen Bereichs des Systems; und
- Figur 7: a)eine schematische perspektivische Darstellung eines erfindungsgemäßen Systems gemäß einer vierten Ausführungsform; b) schematische perspektivische Ansicht eines Teils des Systems im Bereich der Antriebseinheit gemäß einer ersten Variante (b)) und einer zweiten Variante (c)).
- Figur 8: Schematische, teilgeschnitte perspektivische Draufsicht auf ein erfindungsgemäßes System gemäß einer fünften Ausführungsform mit der Betätigungseinheit des Systems im Auslieferungszustand.

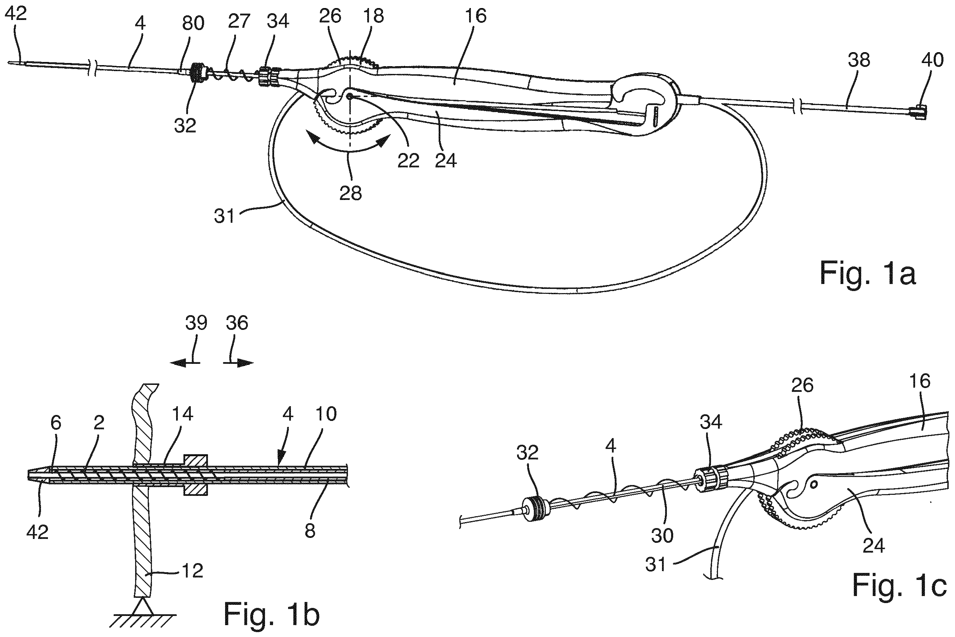

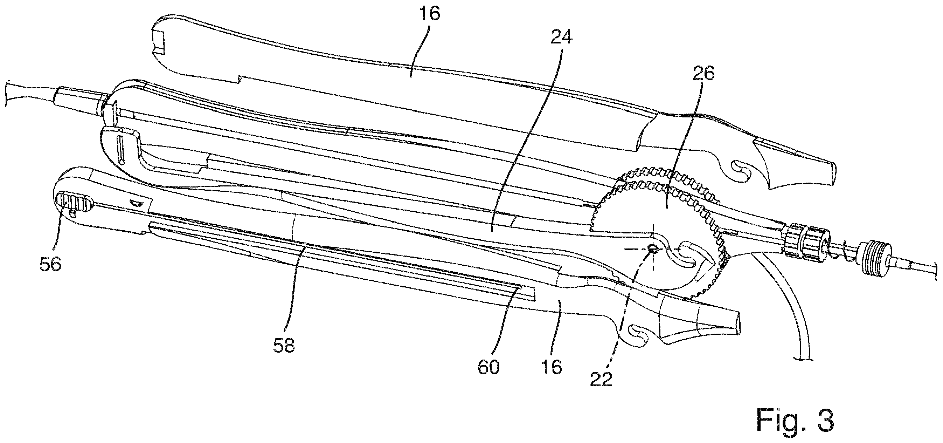

Die Figuren 1 bis 4 zeigen ein erfindungsgemäßes System gemäß einer ersten Ausführungsform. Das System dient dabei zum Einführen und Freisetzen eines selbstexpandierbaren, geflochtenen Stents 2, der in seiner komprimierten Form in Figur 1b zu erkennen ist. Der Stent ist in dieser Form radial verengt und im Vergleich zur selbstexpandierten Form deutlich länger. Ferner ist ein Katheter 4 mit einem Innenschlauch 6, einem Mittelschlauch 8 und einem Außenschlauch 10 dargestellt, wobei sich der Mittelschlauch 8 durch den Außenschlauch 10 erstreckt und sich der Innenschlauch 6 durch den Mittelschlauch 8 erstreckt. Der komprimierte Stent 2 ist in einem distalen Endabschnitt des Katheters 4 radial um den Innenschlauch 6 verlaufend zwischen dem Innenschlauch 6 und dem Außenschlauch 10 radial komprimiert angeordnet, wobei der Mittelschlauch 8 distal am proximalen Ende des komprimierten Stents 2 endet. Der Katheter 4 ist durch eine an einem Blutgefäß 12 angeordnete Schleuse 14 hindurch in das Blutgefäß eingeführt.

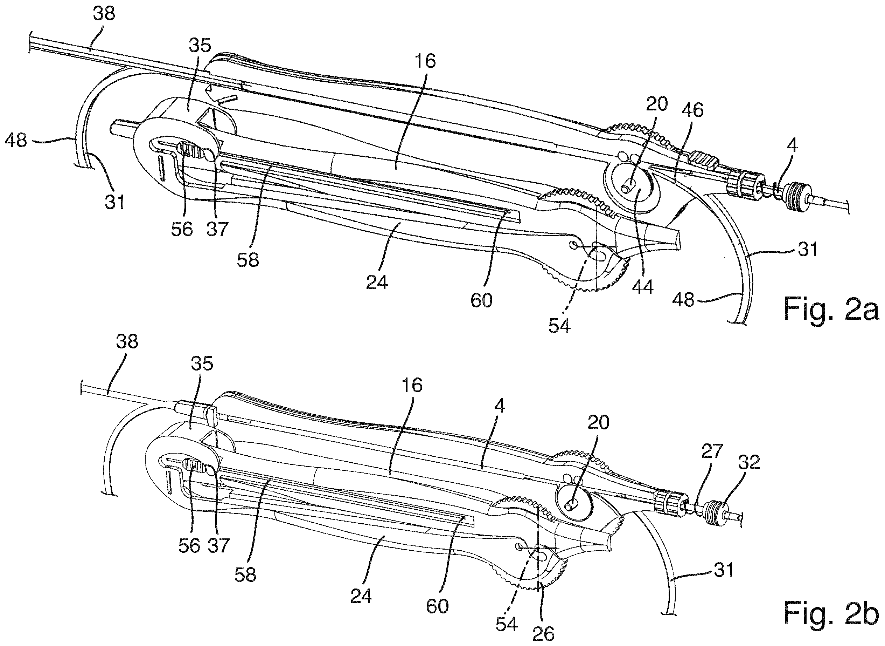

Im proximalen Bereich des Katheters 4, außerhalb des Blutgefäßes 12, ist eine Betätigungseinheit 16 mit einer sich darin befindlichen Antriebseinheit 18 vorgesehen. Die Antriebseinheit 18 umfasst eine in Figur 2 zu erkennende Drehwelle 20, die um eine Drehachse 22 drehbar ist und an einem Lagerabschnitt 24 der Betätigungseinheit 16 drehbar gelagert ist. Ferner ist ein Drehrad 26 zur händischen oder automatisierten Drehung der Drehwelle 20 in Drehrichtung 28 um die Drehachse 22 an der Drehwelle 20 angeordnet.

Zwischen der Antriebseinheit 18 und der Schleuse 14 ist ein als Verbindungsschlauch ausgebildetes Verbindungsmittel 30 angeordnet. Das Verbindungsmittel 30 weist auf seinem distalen Ende einen Befestigungsabschnitt 32 auf, mittels welchem das Verbindungsmittel 30 an der Schleuse 14 befestigt ist. Das Verbindungsmittel 30 verläuft dabei in proximaler Richtung 36 zunächst durch die Antriebseinheit 18 und weiter durch die Betätigungseinheit 16 und mündet in einem bogenförmigen Führungsschlauch 31.

Der Befestigungsabschnitt 32 weist einen Katheterführungsabschnitt 80 auf, der das Innere der Schleuse14 durchgreift. Der Katheter 4 wird folglich durch den Katheterführungsabschnitt 80 des Befestigungsabschnitts 32 in das Blutgefäß 12 eingeführt. Dabei wirkt der Katheterführungsabschnitt 80 gleichzeitig als Friktionsreduzierer. Hierzu ist dieser entweder mit POM oder PTFE beschichtet oder besteht aus einem dieser Materialien bzw. umfasst eines dieser Materialien.

Zwischen dem Befestigungsabschnitt 32 und der Betätigungseinheit 16 ist ein als Spiralfeder ausgebildetes Stabilisierungselement 27 um den Katheter 4 und das Verbindungsmittel 30 angeordnet. Dieses dient insbesondere als Knickschutz gegen ein Ausknicken des Katheters 4 oder auch des Verbindungsmittels 30 bei Druckkräften auf diese.

Der Katheter 4 ist durch eine Öffnung 34 in die Betätigungseinheit 16 eingeführt und darin angeordnet. In proximaler Richtung 36 endet der Außenschlauch 10 vor dem Mittelschlauch 8 und dem Innenschlauch 6. Dabei ist der proximale Endbereich des Außenschlauchs 10 im Bereich der Öffnung 34 an der Betätigungseinheit 16 angeordnet.

Der Innenschlauch 6 ist durch die Betätigungseinheit 16 hindurchgeführt und endet im proximalen Bereich der Betätigungseinheit in einem Führungsschlauch 38, der an seinem proximalen Ende einen Spülanschluss 40 aufweist. An diesem Spülanschluss 40 ist das proximale Ende des Innenschlauchs 6 fluiddicht befestigt.

Über den Spülanschluss 40 kann Flüssigkeit dem distalen Endbereich des Katheters 4 zugeführt werden. Dabei weist der Innenschlauch 6 eine Spitze 42 auf, sodass der Außenschlauch 10 mit dem Innenschlauch 6 derart fluidisch verbindbar ist, dass Fluid durch den Innenschlauch 6 dem distalen Ende des Katheters 4 zuführbar ist und zwischen dem Außenschlauch 10 und dem Mittelschlauch 8 und/oder dem Mittelschlauch 8 und dem Innenschlauch 6 aus dem Katheter 4 abführbar ist.

Um den Mittelschlauch 8 anzutreiben, weist die Antriebswelle 20 ein in Figur 2 zu erkennendes Antriebsrad 44 auf. Dieses kann insbesondere als Zahnrad ausgebildet sein. Hierbei ist einerseits denkbar, dass das Antriebsrad 44 auf eine flexible Zahnstange 46 wirkt, was in Figur 2a dargestellt ist. Dabei ist das proximale Ende der Zahnstange 46 am proximalen Ende des Mittelschlauchs 8 sein. Hierbei ist einerseits denkbar, dass das Antriebsrad 44 auf eine flexible Zahnstange 46 wirkt, was in Figur 2a dargestellt ist. Dabei ist das proximale Ende der Zahnstange 46 am proximalen Ende des Mittelschlauchs 8 befestigt. In distaler Richtung 39 tritt diese Zahnstange 46 aus der Betätigungseinheit 16 aus und mündet in einem zweiten bogenförmigen Führungsschlauch 48. Durch Drehen des Drehrads 26 können sodann die Zahnstange 46 und damit der Mittelschlauch 8 in distaler Richtung bewegt werden.

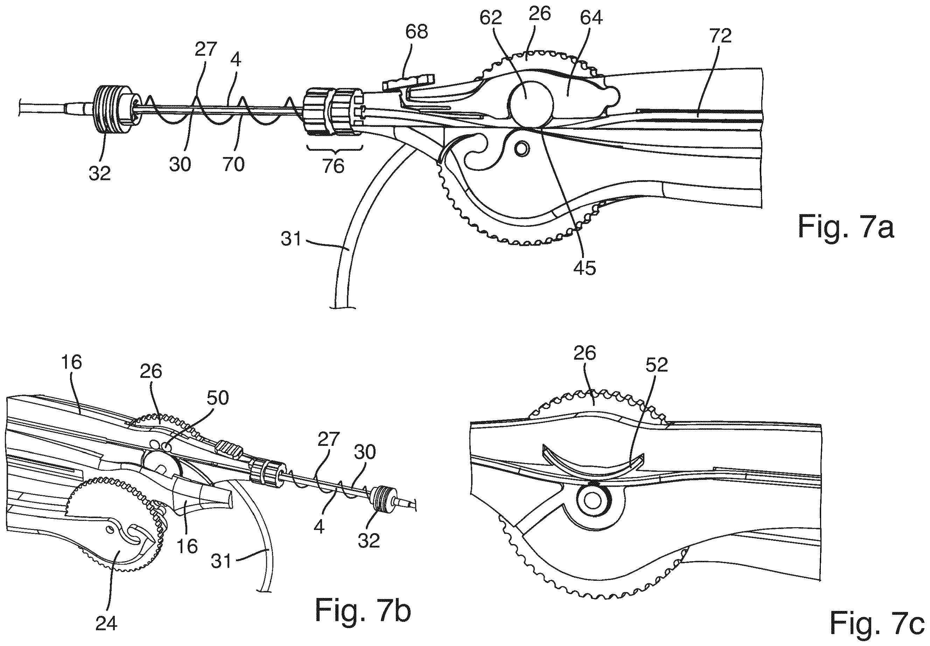

Andererseits ist auch denkbar, dass das Antriebsrad 44, wie in Figur 2b dargestellt, unmittelbar auf den Mittelschlauch 8 wirkt. Um dabei eine zuverlässige Bewegungskopplung zu gewährleisten, kann ein Drückelemente 50, 52 vorgesehen sein, was in den Figuren 7b und 7c zu erkennen ist, und auch für diese erste Ausführungsform des erfindungsgemäßen Systems Anwendung finden kann. Dabei kann das Drückelement beispielsweise als Kugellager 50 oder als Blattfeder 52 ausgebildet sein. Das Drückelement 50, 52 drückt dabei den Mittelschlauch insbesondere unter elastischer Vorspannung gegen das Antriebsrad 44.

Ebenso weist die Antriebswelle ein Antriebsrad 45 (vgl. 5a und 7a) auf, welches entweder mittelbar oder unmittelbar mit dem Verbindungsmittel 30 zusammenwirkt.

Um das Verbindungsmittel 30 vorspannen zu können sowie die Lage der Betätigungseinheit 16 vor der Freisetzung eines Stents 2 verändern zu können, ist der Lagerabschnitt 24 derart bewegbar, dass das Zusammenwirken mit dem Mittelschlauch 8 aufhebbar ist. Dabei ist der Lagerabschnitt 24 um eine Schwenkachse 54 verschwenkbar. Hierzu ist der Lagerabschnitt 26 distalen Bereich der Betätigungseinheit 16 an diese schwenkbar angelenkt. In der verschwenkten Lage, die in Figur 2 gezeigt ist, ist das Drehrad 26 drehbar und die Betätigungseinheit 16 in proximaler Richtung 36 bewegbar, ohne dass gleichzeitig der Mittelschlauch 8 vom Antriebsrad 44 in distaler Richtung 39 bewegt wird.

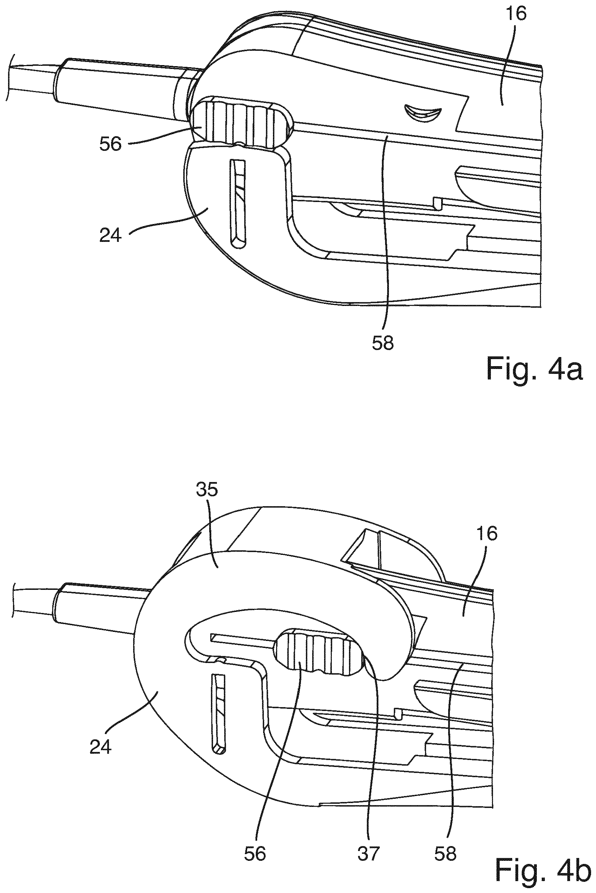

Im proximalen Bereich der Betätigungseinheit 16 ist ein Verlagerungselement 56 vorgesehen, welches am Führungsschlauch 38 befestigt ist. Dieses ist entlang einer Führung 58 im Betätigungselement 16 verlagerbar. In der ersten Lage, die Figur 4a gezeigt ist, liegt das Verlagerungselement in der Führung am proximalen Ende der Führung an und verhindert damit, dass der Lagerabschnitt 26 verschwenkbar ist. Es wird somit eine Bewegungskopplung zwischen Antriebseinheit 18 und Mittelschlauch 8 verhindert. Wenn, wie in Figur 4b gezeigt, das Verlagerungselement 56 in eine zweite Lage verlagert ist, kann der Lagerabschnitt 24 zur Betätigungseinheit 16 hin verschwenkt werden und somit eine Bewegungskopplung zwischen Antriebseinheit 18 und Mittelschlauch 8 hergestellt werden. Hierzu kann der Lagerabschnitt 24 im proximalen Bereich der Betätigungseinheit 16 an dieser mittels einer nicht gezeigten Rastverbindung lösbar befestigt werden. Zum erneuten Schwenken des Lagerabschnitts 26 ist die Rastverbindung zu lösen. Im Gegensatz zur Variante in Figur 4a, weist der Lagerabschnitt 24 gemäß Figur 4b einen Bügel 35 mit einem Anschlag 37 auf, wobei das Verlagerungselement 56 in der zweiten Lage am Anschlag 37 anliegt, sodass der Lagerabschnitt 24 verschwenkbar ist.

Zur Freisetzung des Stents 2 muss folglich zunächst die Rastverbindung hergestellt werden, um somit ein Zusammenwirken der Antriebseinheit 18 mit dem Mittelschlauch 8 zu ermöglichen. Ein Verlagern des Verlagerungselements 56 in distaler Richtung 39 bewirkt zudem, dass die Spitze 42 des Katheters 4 in distaler Richtung 39 verlagert wird und dadurch der Katheter 4 geöffnet wird, sodass insbesondere zu Beginn eine Stentfreisetzung ermöglicht ist.

Zur Freisetzung des Stents 2 wird das Drehrad 26 von einer Bedienperson gemäß Figur 1 entgegen dem Uhrzeigersinn betätigt. Dies bewirkt zweierlei Dinge: Zum einen wird durch das Zusammenwirken des Antriebsrads 31 mit dem Mittelschlauch 8 dieser in distaler Richtung 39 bewegt. Synchron hierzu wird das Verbindungsmittel 30 ebenfalls von einem nicht dargestellten Antriebsrad aus der Betätigungseinheit 16 herausgeführt, was eine Bewegung der Betätigungseinheit 16 sowie des Außenschlauch 10 in proximaler Richtung 36 bewirkt. Der komprimierte Stent 2 ist dabei derart zwischen Außenschlauch 10 und Innenschlauchs 6 angeordnet, dass ein Verschieben des Mittelschlauchs 8 in distale Richtung 39 auch eine Bewegung des Innenschlauchs 6 in distaler Richtung 39 bewirkt. Diese Bewegungskopplung ist dabei so lange vorhanden, bis das Verlagerungselement 56 am als Anschlag 60 ausgebildeten distalen Ende der Führung 58 zur Anlage kommt. Ab diesem Zeitpunkt wird der Innenschlauch 10 sodann ebenfalls in proximaler Richtung 36 zusammen mit der Betätigungseinheit 16 verlagert. Dabei kann beispielsweise ein Stent 2 mit einer Länge von 570 mm freigesetzt werden, sodass der Mittelschlauch 8 um insgesamt 570 mm in distaler Richtung bewegt wird, um den Stent 2 freizusetzen. Das Verlagerungselement 56 und damit der Innenschlauch 6 können hierbei um insbesondere um 200 mm verlagert werden. Die weiteren 370 mm findet keine Bewegungskopplung zwischen Spitze 42 des Innenschlauchs 6 rasch und kontrolliert durch den Stent hindurch aus diesem zurückgezogen werden.

Somit wird nach und nach der Stent 2 freigesetzt, wobei dieser seine Bestimmungsposition beibehält. Die Distanzen der Relativbewegung der Betätigungseinheit 42 sowie des Mittelschlauchs 8 zur Schleuse 14 nach vollständiger Drehung des Drehrads 32 sind dabei unterschiedlich. Dieses Übersetzungsverhältnis kann über die Wahl der Radien des Antriebsrads 44 für den Mittelschlauch 8 sowie des Antriebsrads für das Verbindungsmittel 30 eingestellt werden und kann insbesondere je nach Länge des Stents 2 variieren.

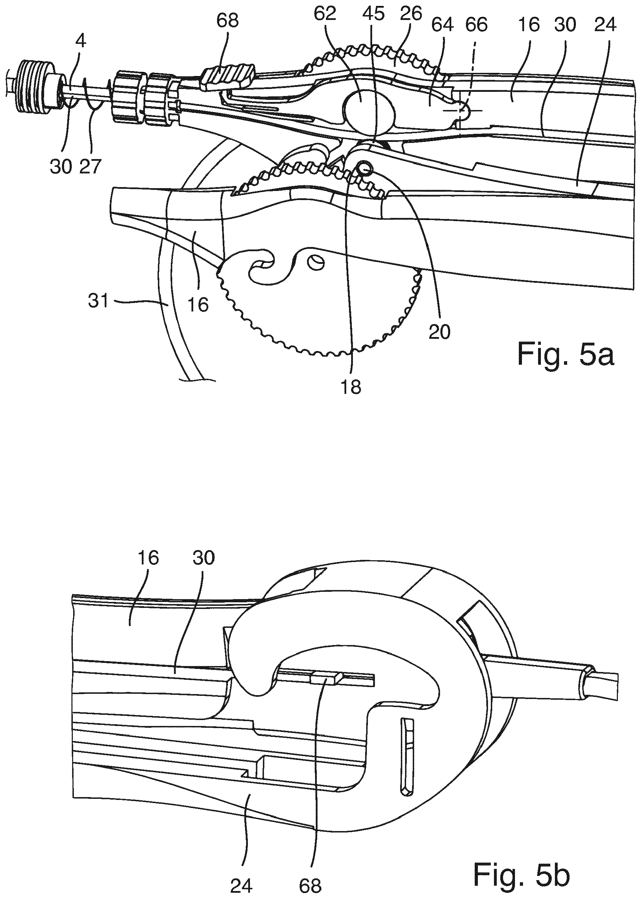

Figur 5 zeigt eine zweite Ausführungsform des erfindungsgemäßen Systems. In Ergänzung zur ersten Ausführungsform ist hierbei ebenfalls die Bewegungskopplung zwischen Verbindungsmittel 30 und der Antriebseinheit 18 aufhebbar. Hierzu ist ein Drückelement 62 an einem Schwenkteil 64 gelagert. Das Schwenkteil 64 ist um eine Schwenkachse 66 verschwenkbar. Gemäß Figur 5a drückt das Drückelement 62 dabei das Verbindungsmittel 30 unter Vorspannung gegen die Antriebseinheit 18 mit dem Antriebsrad 45 für das Verbindungsmittel 30. Zum Verschwenken ist ebenfalls eine Rastverbindung vorgesehen, sodass durch Ziehen an dem Hebel 68 das Schwenkteil 64 an der Betätigungseinheit 16 verrastbar ist, um eine Bewegungskopplung zwischen Antriebseinheit 18 und Verbindungsmittel 30 aufzuheben. Somit kann eine Bedienperson auch manuell die Betätigungseinheit 16 verlagern, ohne, dass ein bestimmtes Übersetzungsverhältnis durch die Antriebseinheit 18 vorgegeben wird.

In diesem Zusammenhang kann vorgesehen sein, dass wie in Figur 5b gezeigt ist, eine Bewegungskopplung zwischen Verbindungsmittel 30 und Innenschlauch 6 vorhanden ist und nicht zwischen Mittelschlauch 8 und Innenschlauchs 6. Um hierbei die Betätigungseinheit 16 vor dem Freisetzen des Stents 2 verlagern zu können, ohne dass dies einen Einfluss auf den Innenschlauch hätte, ist ebenfalls eine Bewegungsentkopplung zwischen Verbindungsmittel 30 und Innenschlauchs 6 vorhanden. Hierzu ist ein Clips-Element 68 am proximalen Endbereich der Betätigungseinheit 16 vorgesehen, um somit entweder eine Bewegungskopplung zwischen Verbindungsmittel 30 und Innenschlauchs 6 herzustellen oder aufzuheben. Hierbei kann vorgesehen sein, dass das Übersetzungsverhältnis der Antriebsräder 44, 45 so gewählt ist, dass das Clips-Element 68 dann am distalen Ende der Betätigungseinheit 16 angelangt, wenn der Stent 2 freigesetzt ist.

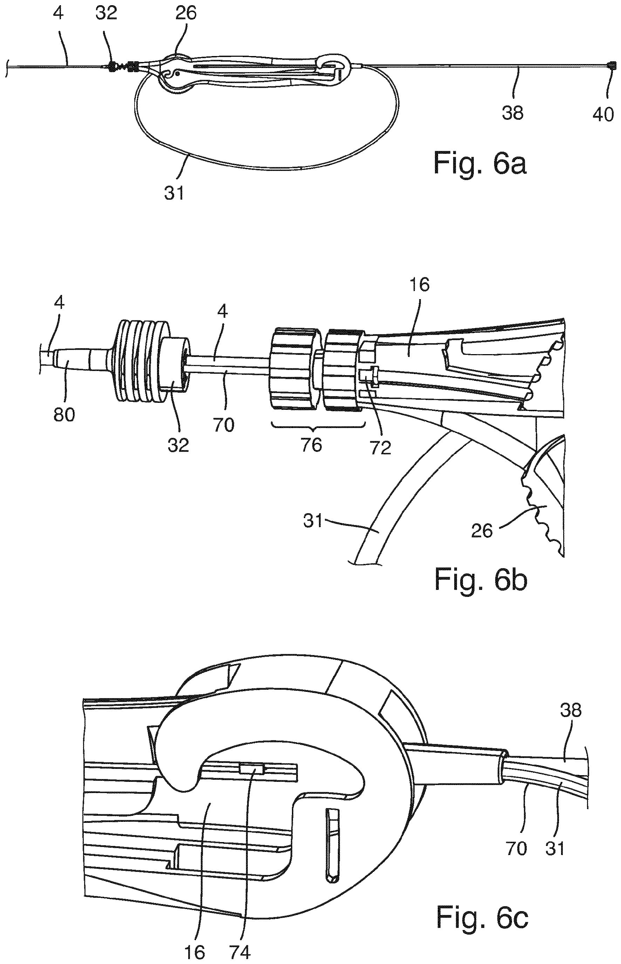

Figur 6 zeigt eine weitere Ausführungsform des erfindungsgemäßen Systems. Auch hier ist im Gegensatz zur ersten Ausführungsform gemäß den Figuren 1-4 keine Bewegungskopplung zwischen Mittelschlauch 8 und Innenschlauch 6 vorhanden. Zudem ist keine Spitzenöffnung vor dem Stentfreisetzen vorgesehen.

Stattdessen ist das Verbindungsmittel 30 zweiteilig aufgebaut und umfasst einen als Verbindungsdraht ausgebildeten ersten Abschnitt 70 und einen als Verbindungsschlauch ausgebildeten zweiten Abschnitt 72. Der erste Abschnitt 70 ist dabei über den Befestigungsabschnitt 32 an der Schleuse 14 befestigt. Der zweite Abschnitt 72 ist über ein Befestigungsmittel 74 mit dem Innenschlauch 6 verbunden. Der erste Abschnitt 70 mündet dabei in proximaler Richtung 36 im zweiten Abschnitt 72 und ist durch diesen hindurchgeführt. Im proximalen Bereich der Betätigungseinheit 16 tritt der erste Abschnitt 70 sodann in den Führungsschlauch 31 ein. Ferner wirkt der zweite Abschnitt 72 mit dem Antriebsrad der Antriebseinheit 18 zusammen.

Im distalen Endbereich der Betätigungseinheit 16 ist eine Spannvorrichtung 76 vorgesehen, die entweder so eingestellt ist, dass die beiden Abschnitte 70,72 gegeneinander verlagerbar sind oder aneinander befestigt sind. In einem ersten Betriebszustand sind diese gegeneinander verlagerbar, um die Betätigungseinheit 16 positionieren zu können, ohne dass dies eine Verlagerung des Innenschlauchs 6 bewirkt. In einem zweiten Betriebszustand, nämlich insbesondere dann, wenn der Stent 2 freigesetzt wird, sind die beiden Abschnitte 70,72 mittels der Spannvorrichtung 76 gegeneinander verspannt und damit aneinander befestigt und bewegungsgekoppelt. Eine Bewegung des Verbindungsmittels 30 bewirkt somit eine Verlagerung der Betätigungseinheit 16 relativ zum Innenschlauch 6, sodass dieser seine Position beibehält, während der Mittelschlauch 8 und der Außenschlauch 10 relativ dazu verlagert werden. Auch hierbei kann vorgesehen sein, dass das Übersetzungsverhältnis der Antriebsräder 44, 45 so gewählt ist, dass die Spannvorrichtung 76 dann am distalen Ende der Betätigungseinheit 16 angelangt, wenn der Stent 2 freigesetzt ist.

Figur 7 zeigt eine weitere Ausführungsform des erfindungsgemäßen Systems. In Weiterbildung des Systems gemäß Figur 6, ist dabei die Bewegungskopplung zwischen Verbindungsmittel 30 und der Antriebseinheit 18 aufhebbar. Hierzu ist, wie in Figur 5 bereits dargelegt, wiederum ein Schwenkteil 64 mit einem Drückelement 62 vorgesehen.

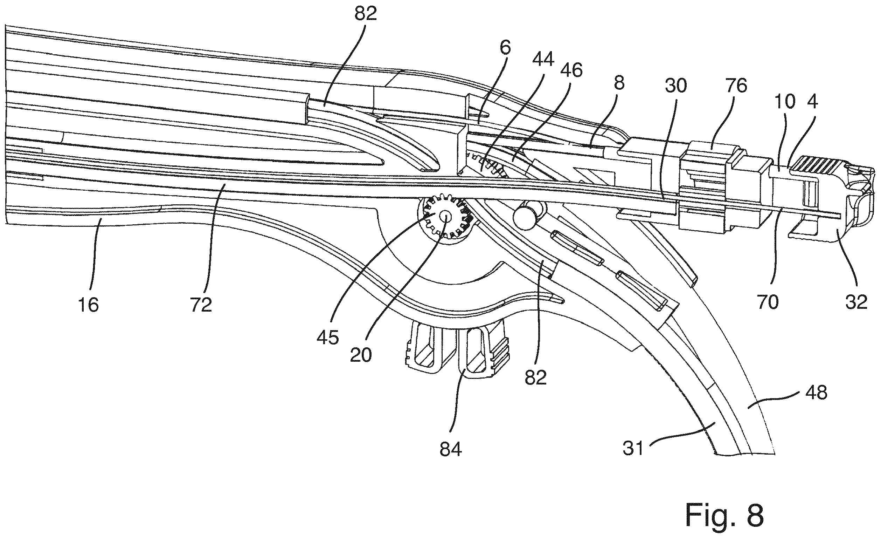

Figur 8a zeigt eine weitere Ausführungsform des erfindungsgemäßen Systems. In Weiterbildung der Ausführungsform gemäß Figur 6 ist der Innenschlauch 6 hierbei zwar fluiddicht, aber nicht lagefest mit dem Führungsschlauch 38 befestigt. Ferner ist der Innenschlauch nicht unmittelbar mit dem Verbindungsmittel 30 bewegungsgekoppelt. Zudem wird der Mittelschlauch 8 von der Zahnstange 46 angetrieben, ähnlich dem Ausführungsbeispiel gemäß Figur 2a.

Stattdessen wird der Innenschlauch 6 innerhalb der Betätigungseinheit 16 von einem geschlitzten Antriebsschlauch 82 bewegungsgekoppelt angetrieben. Der Antriebsschlauch 82 wird von dem Antriebsrad 45 für das Verbindungsmittel 30 betätigt. Denkbar wäre allerdings auch, dass ein separates Antriebsrad vorhanden ist. Sodann mündet der Antriebsschlauch 82 im bogenförmigen Führungsschlauch 31. Denkbar wäre auch, eine flexible Zahnstange zum Antreiben des Innenschlauchs 6 zu verwenden, ähnlich der Zahnstange 46 für den Mittelschlauch 8. Wie in Figur 8 deutlich zu erkennen ist, ist der Mittelschlauch 8 jedenfalls abschnittsweise derart geschlitzt, dass der Innenschlauch 6 innerhalb der Betätigungseinheit 16 nach dem Verlassen des Antriebsschlauchs 82 in den Mittelschlauch 8 hinein geführt wird.

Figur 8 zeigt die Betätigungseinheit 16 im Auslieferungszustand, also vor Verwendung im erfindungsgemäßen System. Hierbei ist ein Blockierelement 84 an der Betätigungseinheit 16 verclipst. Mit einem nicht gezeigten Zahnstangenabschnitt wirkt das Blockierelement 84 auf das Antriebsrad 44 und zwar derart, dass eine Drehung des Drehrads 26 und damit der Drehwelle 20 vollständig unterbunden ist. Um die Betätigungseinheit 16 sodann im System einsetzen zu können, wird das Blockierlement 84 zusammengedrückt, sodass die Clipverbindung gelöst wird. Sodann kann das Drehrad 26 zur Stentfreisetzung gedreht werden.

Bei allen Ausführungsformen kann vorgesehen sein, dass der Mittelschlauch 8 ein diesen verlängerndes Stenteingreifelement aufweist, welches derart in den Stent 2 eingreift, dass auch ein Zurückziehen des Stents 2 in den Katheter 4 ermöglicht ist, solange dieser noch nicht vollständig freigesetzt ist. Um den Stent 2 zurückzuziehen muss folglich lediglich das Drehrad 26 in umgekehrter Drehrichtung 28 gedreht werden.

Insgesamt wird mittels der Erfindung die präzise Freisetzung eines Stents 2 in einem Blutgefäß 12 ermöglicht. Insbesondere kann zudem das System auch einhändig, sowohl links- als auch rechtshändig bedient werden.

## Patentansprüche

1. System zum Einführen eines komprimierten, selbstexpandierbaren Stents (2) durch eine an einem Blutgefäß (12) angeordnete Schleuse (14) hindurch in das Blutgefäß (12) und zur Freisetzung des Stents (2) in dem Blutgefäß (12), das System umfassend eine Schleuse (14) und einen Katheter (4), welcher einen Innenschlauch (6), einen Mittelschlauch (8) und einen Außenschlauch (10) umfasst, wobei der Stent (2) in einem distalen Endabschnitt des Katheters (4) zwischen dem Innenschlauch (6) und dem Außenschlauch (10) angeordnet ist und der Mittelschlauch (8) distal am proximalen Ende des Stents (2) endet, und mit einer im proximalen Bereich des Katheters (4) zur Freisetzung des Stents (2) vorgesehenen Betätigungseinheit (16) mit einer Antriebseinheit (18), **dadurch gekennzeichnet, dass** zwischen der Antriebseinheit (18) und der Schleuse (14) ein Verbindungsmittel (30) vorgesehen ist, wobei die Antriebseinheit (18) derart mit dem Mittelschlauch (8) und dem Verbindungsmittel (30) zusammenwirkt, dass bei deren Betätigung zum einen der Mittelschlauch (8) in distaler Richtung (39) verschoben wird und zum anderen die Betätigungseinheit (16) samt daran befestigtem Außenschlauch (10) in proximaler Richtung (36) verschoben wird, und wobei der Innenschlauch (6) derart mit dem Mittelschlauch (8) und/oder dem Verbindungsmittel (30) und/oder der Antriebseinheit (18) zusammenwirkt, dass dieser zumindest bedingt relativ zur Betätigungseinheit (16) verlagert wird.

2. System nach Anspruch 1, **dadurch gekennzeichnet, dass** der Innenschlauch (6) mit dem Mittelschlauch (8) bewegungsgekoppelt ist, wobei der komprimierte Stent (2) derart am Innenschlauch (6) kraftschlüssig angeordnet ist, dass dieser beim Verschieben des Mittelschlauchs (8) und damit des Stents (2) in distaler Richtung (39) ebenfalls in distaler Richtung (39) verschoben wird.

3. System nach einem der vorhergehenden Ansprüche, **dadurch gekennzeichnet, dass** die Betätigungseinheit (16) eine Bewegungsbegrenzung (60) für den Innenschlauch (6) aufweist, sodass eine Bewegung des Innenschlauchs (6) relativ zur Betätigungseinheit (16) lediglich bedingt ermöglicht ist.

4. System nach Anspruch 1, **dadurch gekennzeichnet, dass** der Innenschlauch (6) am Verbindungsmittel (30) befestigt ist, sodass die Betätigungseinheit (16) samt Außenschlauch (10) beim Verschieben derselben in proximaler Richtung (36) relativ zum Innenschlauch (6) bewegt wird.

5. System nach Anspruch 4, **dadurch gekennzeichnet, dass** das Verbindungsmittel (30) einen ersten Abschnitt (70) und einen zweiten Abschnitt (72) umfasst, wobei der erste Abschnitt (70) an der Schleuse (14) befestigt oder befestigbar ist, wobei der zweite Abschnitt (72) am Innenschlauch (6) befestigt ist und mit der Antriebseinheit (18) zusammenwirkt, und wobei die beiden Abschnitte (70, 72) in einem ersten Betriebszustand relativ zueinander verlagerbar sind und in einem zweiten Betriebszustand aneinander befestigt sind.

6. System nach Anspruch 1, **dadurch gekennzeichnet, dass** der Innenschlauch (6) mittels der Antriebseinheit (18) relativ zur Betätigungseinheit (16) verlagerbar ist.

7. System nach einem der vorhergehenden Ansprüche, **dadurch gekennzeichnet, dass** das Zusammenwirken der Antriebseinheit (18) mit dem Mittelschlauch (8) und/oder dem Verbindungsmittel (30) aufhebbar ist.

8. System nach Anspruch 7, **dadurch gekennzeichnet, dass** die Antriebseinheit (18) an einem Lagerabschnitt (24) angeordnet ist, wobei der Lagerabschnitt (24) verschwenkbar ist, sodass das Zusammenwirken der Antriebseinheit (18) mit dem Mittelschlauch (8) und/oder dem Verbindungsmittel (30) aufhebbar ist.

9. System nach Anspruch 8, **dadurch gekennzeichnet, dass** ein mit dem Innenschlauch (6) bewegungsgekoppeltes Verlagerungselement (56) vorhanden ist, wobei das Verlagerungselement (56) in einer ersten Lage so an der Betätigungseinheit (16) angeordnet ist, dass der Lagerabschnitt (24) so verschwenkt ist, dass das Zusammenwirken der Antriebseinheit (18) mit dem Mittelschlauch (8) und/oder dem Verbindungsmittel (30) aufgehoben ist, und dass das Verlagerungselement (56) in einer zweiten Lage so entlang der Betätigungseinheit (16) in distaler Richtung (39) verlagert ist, dass der Lagerabschnitt (24) verschwenkbar ist.

10. System nach einem der vorhergehenden Ansprüche, **dadurch gekennzeichnet, dass** der Innenschlauch (6) am distalen Ende eine Spitze (42) aufweist, wobei die Spitze (42) den Katheter (4) fluiddicht verschließt, wenn das Verlagerungselement (56) in der ersten Lage ist, und wobei der Katheter (4) geöffnet ist, wenn das Verlagerungselement (56) in der zweiten Lage ist.

11. System nach Anspruch 9 oder 10, **dadurch gekennzeichnet, dass** der Lagerabschnitt (24) einen Bügel (35) mit einem Anschlag (37) aufweist, wobei das Verlagerungselement (56) in der zweiten Lage am Anschlag (37) anliegt, und sodann der Lagerabschnitt (24) verschwenkbar ist.

12. System nach einem der Ansprüche 9 oder 10 oder 11, **dadurch gekennzeichnet, dass** das proximale Ende des Innenschlauchs (6) an einem Führungsschlauch (38) mit einem Spülanschluss (40) befestigt ist, wobei der Führungsschlauch (38) das Verlagerungselement (56) aufweist.

13. System nach einem der vorhergehenden Ansprüche, **dadurch gekennzeichnet, dass** die Betätigungseinheit (16) ein Drückelement (50, 52, 62) aufweist, das den Mittelschlauch (8) und/oder das Verbindungsmittel (30) zur Bewegungskopplung gegen die Antriebseinheit (18) drückt.

14. System nach Anspruch 13, **dadurch gekennzeichnet, dass** das Drückelement (62) verschwenkbar ist, sodass das Zusammenwirken der Antriebseinheit (18) mit dem Mittelschlauch (8) und/oder dem Verbindungsmittel (30) aufhebbar ist.

15. System nach einem der vorhergehenden Ansprüche, **dadurch gekennzeichnet, dass** das Verbindungsmittel (30) an seinem distalen Ende einen Befestigungsabschnitt (32) aufweist, mittels welchem das Verbindungsmittel (30) an der Schleuse (14) befestigt oder befestigbar ist, wobei der Befestigungsabschnitt (32) einen die Schleuse (14) im befestigten Zustand durchgreifenden Katheterführungsabschnitt (80) aufweist, in dem der Katheter (4) geführt ist.

16. System nach einem der vorhergehenden Ansprüche, **dadurch gekennzeichnet, dass** zwischen der Schleuse (14) und dem Befestigungsabschnitt (32) ein um das Verbindungsmittel (30) und/oder den Katheter (4) verlaufendes Stabilisierungselement (27), insbesondere eine Spiralfeder oder ein geflochtenes Element, zur Verhinderung des Durchbiegens des Verbindungsmittels (30) und/oder des Katheters (4) vorgesehen ist.

## Claims

1. System for inserting a compressed, self-expandable stent (2) through a sluice (14) arranged on a blood vessel (12) into the blood vessel (12) and for releasing the stent (2) in the blood vessel (12), the system comprising a sluice (14) and a catheter (4), which comprises an inner tube (6), a middle tube (8) and an outer tube (10), the stent (2) being arranged in a distal end portion of the catheter (4) between the inner tube (6) and the outer tube (10) and the middle tube (8) ending distally at the proximal end of the stent (2), and comprising an actuating unit (16) which is provided in the proximal region of the catheter (4) for releasing the stent (2) and has a drive unit (18), **characterized in that** a connecting means (30) is provided between the drive unit (18) and the sluice (14), the drive unit (18) interacting with the middle tube (8) and the connecting means (30) such that, when the drive unit is actuated, the middle tube (8) is shifted in the distal direction (39) and the actuating unit (16) together with the outer tube (10) attached thereto is shifted in the proximal direction (36), and the inner tube (6) interacting with the middle tube (8) and/or the connecting means (30) and/or the drive unit (18) such that it is displaced at least to a limited extent relative to the actuating unit (16).

2. System according to claim 1, **characterized in that** the inner tube (6) is movement-coupled to the middle tube (8), the compressed stent (2) being arranged frictionally on the inner tube (6) such that it is also shifted in the distal direction (39) when the middle tube (8) and thus the stent (2) is shifted in the distal direction (39).

3. System according to any of the preceding claims, **characterized in that** the actuating unit (16) has a movement-limiting means (60) for the inner tube (6), such that moving the inner tube (6) relative to the actuating unit (16) is only possible to a limited extent.

4. System according to claim 1, **characterized in that** the inner tube (6) is attached to the connecting means (30), such that the actuating unit (16) together with the outer tube (10) is moved relative to the inner tube (6) when said outer tube is shifted in the proximal direction (36).

5. System according to claim 4, **characterized in that** the connecting means (30) comprises a first portion (70) and a second portion (72), the first portion (70) being attached or attachable to the sluice (14), the second portion (72) being attached to the inner tube (6) and interacting with the drive unit (18), and it being possible for the two portions (70, 72) to be displaced relative to one another in a first operating state and said two portions being attached to one another in a second operating state.

6. System according to claim 1, **characterized in that** the inner tube (6) can be displaced relative to the actuating unit (16) by means of the drive unit (18).

7. System according to any of the preceding claims, **characterized in that** the interaction of the drive unit (18) with the middle tube (8) and/or the connecting means (30) can be released.

8. System according to claim 7, **characterized in that** the drive unit (18) is arranged on a bearing portion (24), the bearing portion (24) being pivotable, such that the interaction of the drive unit (18) with the middle tube (8) and/or the connecting means (30) can be released.

9. System according to claim 8, **characterized in that** there is a displacement element (56) which is movement-coupled to the inner tube (6), the displacement element (56) being arranged on the actuating unit (16) in a first position such that the bearing portion (24) is pivoted such that the interaction of the drive unit (18) with the middle tube (8) and/or the connecting means (30) is released, and **in that** the displacement element (56) is displaced along the actuating unit (16) in the distal direction (39) in a second position such that the bearing portion (24) is pivotable.

10. System according to any of the preceding claims, **characterized in that** the inner tube (6) has a tip (42) at the distal end, the tip (42) closing the catheter (4) in a fluid-tight manner when the displacement element (56) is in the first position, and the catheter (4) being opened when the displacement element (56) is in the second position.

11. System according to claim 9 or 10, **characterized in that** the bearing portion (24) has a bracket (35) having a stop (37), the displacement element (56) abutting the stop (37) in the second position, and then the bearing portion (24) being pivotable.

12. System according to any of claims 9 or 10 or 11, **characterized in that** the proximal end of the inner tube (6) is attached to a guide tube (38) having a flushing connection (40), the guide tube (38) having the displacement element (56).

13. System according to any of the preceding claims, **characterized in that** the actuating unit (16) has a pressing element (50, 52, 62) which presses the middle tube (8) and/or the connecting means (30) against the drive unit (18) for movement coupling.

14. System according to claim 13, **characterized in that** the pressing element (62) is pivotable, such that the interaction of the drive unit (18) with the middle tube (8) and/or the connecting means (30) can be released.

15. System according to any of the preceding claims, **characterized in that** the connecting means (30) has an attaching portion (32) at the distal end thereof, by means of which the connecting means (30) is attached or attachable to the sluice (14), the attaching portion (32) having a catheter guide portion (80) which passes through the sluice (14) in the attached state and in which the catheter (4) is guided.

16. System according to any of the preceding claims, **characterized in that** a stabilizing element (27) which extends around the connecting means (30) and/or the catheter (4), in particular a coil spring or a braided element, is provided between the sluice (14) and the attaching portion (32) in order to prevent the connecting means (30) and/or the catheter (4) from bending.

## Revendications

1. Système pour introduire un stent (2) comprimé, auto-expansible, à travers une écluse (14) disposée sur un vaisseau sanguin (12), dans le vaisseau sanguin (12) et pour libérer le stent (2) dans le vaisseau sanguin (12), le système comprenant une écluse (14) et un cathéter (4), lequel comprend un tuyau intérieur (6), un tuyau central (8) et un tuyau extérieur (10), dans lequel le stent (2) est disposé dans une section d'extrémité distale du cathéter (4) entre le tuyau intérieur (6) et le tuyau extérieur (10) et le tuyau central (8) se termine de manière distale à l'extrémité proximale du stent (2), et avec une unité d'actionnement (16), prévue dans la zone proximale du cathéter (4) pour libérer le stent (2), avec une unité d'entraînement (18), **caractérisé en ce qu'**un moyen de liaison (30) est prévu entre l'unité d'entraînement (18) et l'écluse (14), dans lequel l'unité d'entraînement (18) coopère avec le tuyau central (8) et le moyen de liaison (30) de telle sorte que, lors de son actionnement, d'une part le tuyau central (8) est décalé dans la direction distale (39) et d'autre part l'unité d'actionnement (16), y compris le tuyau extérieur (10) fixé à celle-ci, est décalée dans la direction proximale (36), et dans lequel le tuyau intérieur (6) coopère avec le tuyau central (8) et/ou le moyen de liaison (30) et/ou l'unité d'entraînement (18) de telle sorte que celui-ci est déplacé au moins sous certaines réserves par rapport à l'unité d'actionnement (16).

2. Système selon la revendication 1, **caractérisé en ce que** le tuyau intérieur (6) est accouplé en mouvement au tuyau central (8), dans lequel le stent (2) comprimé est disposé à force sur le tuyau intérieur (6) de telle sorte que celui-ci, lors du décalage du tuyau central (8) et donc du stent (2) dans la direction distale (39), est également amené à se décaler dans la direction distale (39).

3. Système selon l'une quelconque des revendications précédentes, **caractérisé en ce que** l'unité d'actionnement (16) présente une limitation de mouvement (60) pour le tuyau intérieur (6), de sorte qu'un mouvement du tuyau intérieur (6) par rapport à l'unité d'actionnement (16) est permis uniquement sous certaines réserves.

4. Système selon la revendication 1, **caractérisé en ce que** le tuyau intérieur (6) est fixé au moyen de liaison (30), de sorte que l'unité d'actionnement (16), y compris le tuyau extérieur (10), est déplacée dans la direction proximale (36) par rapport au tuyau intérieur (6) lors du décalage de celle-ci.

5. Système selon la revendication 4, **caractérisé en ce que** le moyen de liaison (30) comprend une première section (70) et une deuxième section (72), dans lequel la première section (70) est fixée ou peut être fixée à l'écluse (14), dans lequel la deuxième section (72) est fixée au tuyau intérieur (6) et coopère avec l'unité d'entraînement (18), et dans lequel les deux sections (70, 72) peuvent être déplacées l'une par rapport à l'autre dans un premier état de fonctionnement et sont fixées l'une sur l'autre dans un deuxième état de fonctionnement.

6. Système selon la revendication 1, **caractérisé en ce que** le tuyau intérieur (6) peut être déplacé par rapport à l'unité d'actionnement (16) au moyen de l'unité d'entraînement (18).

7. Système selon l'une quelconque des revendications précédentes, **caractérisé en ce que** la coopération de l'unité d'entraînement (18) avec le tuyau central (8) et/ou le moyen de liaison (30) peut être supprimée.

8. Système selon la revendication 7, **caractérisé en ce que** l'unité d'entraînement (18) est disposée sur une section de support (24), dans lequel la section de support (24) est pivotante, de sorte que la coopération de l'unité d'entraînement (18) avec le tuyau central (8) et/ou le moyen de liaison (30) peut être supprimée.

9. Système selon la revendication 8, **caractérisé en ce qu'**un élément de déplacement (56) accouplé en mouvement au tuyau intérieur (6) est présent, dans lequel l'élément de déplacement (56) est disposé dans une première position sur l'unité d'actionnement (16) de sorte que la section de support (24) est amenée à pivoter, de sorte que la coopération de l'unité d'entraînement (18) avec le tuyau central (8) et/ou le moyen de liaison (30) est supprimée, et que l'élément de déplacement (56) est déplacé dans une deuxième position le long de l'unité d'actionnement (16) dans la direction distale (39), de sorte que la section de support (24) peut être amenée à pivoter.

10. Système selon l'une quelconque des revendications précédentes, **caractérisé en ce que** le tuyau intérieur (6) présente à l'extrémité distale une pointe (42), dans lequel la pointe (42) ferme le cathéter (4) de manière étanche au fluide, lorsque l'élément de déplacement (56) est dans la première position, et dans lequel le cathéter (4) est ouvert lorsque l'élément de déplacement (56) est dans la deuxième position.

11. Système selon la revendication 9 ou 10, **caractérisé en ce que** la section de support (24) présente un étrier (35) avec une butée (37), dans lequel l'élément de déplacement (56), dans la deuxième position, s'applique contre la butée (37), et ensuite la section de support (24) peut pivoter.

12. Système selon l'une quelconque des revendications 9 ou 10 ou 11, **caractérisé en ce que** l'extrémité proximale du tuyau intérieur (6) est fixée à un tuyau de guidage (38) avec un raccord de rinçage (40), dans lequel le tuyau de guidage (38) présente l'élément de déplacement (56).

13. Système selon l'une quelconque des revendications précédentes, **caractérisé en ce que** l'unité d'actionnement (16) présente un élément de pression (50, 52, 62), qui presse le tuyau central (8) et/ou le moyen de liaison (30) aux fins d'accouplement en mouvement contre l'unité d'entraînement (18).

14. Système selon la revendication 13, **caractérisé en ce que** l'élément de pression (62) peut être amené à pivoter, de sorte que la coopération de l'unité d'entraînement (18) avec le tuyau central (8) et/ou le moyen de liaison (30) peut être supprimée.

15. Système selon l'une quelconque des revendications précédentes, **caractérisé en ce que** le moyen de liaison (30) présente à son extrémité distale une section de fixation (32), au moyen de laquelle le moyen de liaison (30) est fixé ou peut être fixé à l'écluse (14), dans lequel la section de fixation (32) présente une section de guidage de cathéter (80), traversant l'écluse (14) dans l'état fixé, dans laquelle le cathéter (4) est guidé.

16. Système selon l'une quelconque des revendications précédentes, **caractérisé en ce qu'**un élément de stabilisation (27) s'étendant autour du moyen de liaison (30) et/ou du cathéter (4), en particulier un ressort spiral ou un élément tressé, destiné à empêcher la flexion du moyen de liaison (30) et/ou du cathéter (4), est prévu entre l'écluse (14) et la section de fixation (32).
